(19) Europäisches Patentamt
European Patent Office
Office européen des brevets

(11) EP 4 250 309 A1

(12) EUROPEAN PATENT APPLICATION

(43) Date of publication:
27.09.2023 Bulletin 2023/39

(21) Application number: 22193347.6

(22) Date of filing: 31.08.2022

(51) International Patent Classification (IPC):
*G16H 20/10* (2018.01) *G16H 50/50* (2018.01)

(52) Cooperative Patent Classification (CPC):
G16H 20/10; G16H 50/50

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 23.03.2022 IN 202241016187

(71) Applicant: Ansys, Inc.
Canonsburg, PA 15317 (US)

(72) Inventors:
- PUNEKAR, Hemant
  Canonsburg, 15317 (US)
- GOHEL, Shitanshu
  Canonsburg, 15317 (US)

(74) Representative: Paustian & Partner Patentanwälte mbB
Oberanger 32
80331 München (DE)

# (54) SYSTEM AND METHOD OF DETERMINING RESPIRATORY PARTICLE DEPOSITION IN A LUNG

(57) A method and a system of determining respiratory particle deposition in a lung is described. A single conduit model having conduit generations corresponding to generations of the lung is generated. Computational fluid dynamics simulations of respiratory particle delivery to an N conduit generation and an N+1 conduit generation of the single conduit model are performed to determine a mass of respiratory particles leaving the N conduit generation and a mass of respiratory particles deposited in the N+1 conduit generation. The mass deposited in the N+1 conduit generation is scaled up by a scaling factor, the scaling factor being based on the mass of respiratory particles leaving the N conduit generation, to determine a total mass of respiratory particles deposited in a generation of the lung corresponding to the N+1 conduit generation. Other embodiments are also described and claimed.

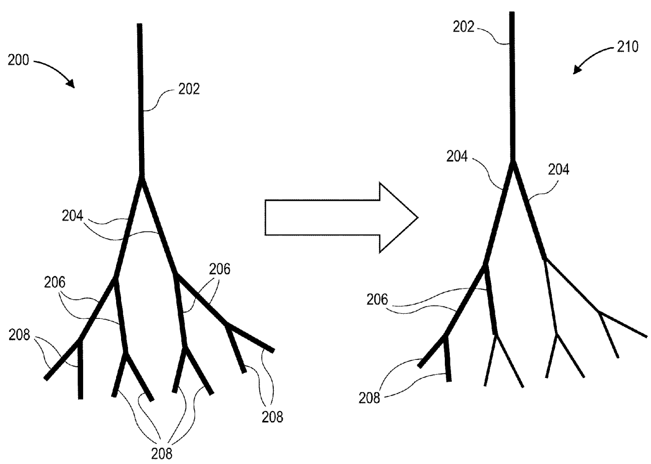


FIG. 2

EP 4 250 309 A1

## Description

The present application claims priority to and the benefit of Indian Patent Application No. 202241016187, filed on March 23, 2022, the disclosure of which is hereby incorporated by reference in its entirety.

## FIELD

The present disclosure relates to methods and systems of modeling particle delivery to anatomical structures. More specifically, the present disclosure relates to methods and systems of modeling respiratory particle deposition in a lung.

## BACKGROUND INFORMATION

Respiratory diseases, such as asthma, place many millions of people at risk around the world. Some respiratory diseases can be managed using therapeutic drugs. The drugs can be delivered to a lung in respiratory particles, e.g., via devices such as an inhaler. Regulatory approval of such drugs and devices traditionally require costly and time-consuming clinical trials. More recently, however, in-silico approaches to simulating the delivery of respiratory particles has been accepted by regulatory bodies as reliable evidence of efficacy to support regulatory approval of respiratory drugs and devices. Beneficially, in-silico approaches can cost less than one tenth of traditional clinical trials.

In-silico approaches use computer simulations of respiratory particle delivery to the lung. Human lungs typically have twenty-three generations of branching, terminating in alveoli that exchange oxygen and carbon dioxide between blood and inhaled air. Simulation of drug delivery to the generations of the lung can be performed by computational fluid dynamics methodologies and 1-dimensional algebraic models. Drug deposition in the lung can be calculated by the simulations, and used to evaluate the efficacy of drug delivery.

Existing in-silico approaches to simulating delivery of respiratory particles utilize computational fluid dynamics (CFD) simulations to model delivery up to a thirteenth generation of the lung. CFD simulations of higher generations, e.g., from the thirteenth to twenty-third generations, however, is computationally prohibitive because there are over sixteen million air passages in the higher generations. Accordingly, analytical models, such as 1-dimensional algebraic models, are used to calculate drug deposition in the higher generations. Such analytical methods, however, have reduced accuracy as compared to CFD simulations.

## SUMMARY

A method and system of modeling respiratory particle deposition in a lung is provided. The method and system provides an accurate and computationally efficient CFD modeling process for determining respiratory drug deposition in the higher generations of the lung. In an embodiment, the method includes generating a single conduit model. The single conduit model has conduit generations representing portions of corresponding generations of a lung. More particularly, the portions include at most two branches of the corresponding generations of the lung. Accordingly, the single conduit model represents a continuous conduit of the full lung geometry, and up to one branch per generation extending therefrom.

The method includes performing a computational fluid dynamics (CFD) simulation of respiratory particle delivery to the single conduit model. The CFD simulation can include transient air flow modeling and particle deposition modeling to determine particle inflow mass, deposition, and outflow mass of a current, or "N," conduit generation. For example, the CFD simulation can include an operation to simulate respiratory particle delivery to the N conduit generation, and the operation can include a calculation to determine a mass of respiratory particles leaving the N conduit generation.

The CFD simulation of respiratory particle delivery to the single conduit model can include an operation to simulate respiratory particle delivery to a next, or "N+1," conduit generation of the single conduit model. The N+1 conduit generation can be adjacent to and connected to the N conduit generation. The CFD simulation can include transient air flow modeling and particle deposition modeling to determine particle inflow mass, deposition, and outflow mass of the N+1 conduit generation. For example, the operation of the CFD simulation can include a calculation to determine a mass of respiratory particles deposited in the N+1 conduit generation.

The method includes scaling up the deposited mass determined by the CFD simulation to determine a total mass of respiratory particles deposited in a generation of the lung corresponding to the N+1 conduit generation. The determination is based on the mass of respiratory particles deposited in the N+1 conduit generation and a scaling factor for the N+1 conduit generation. The scaling factor is based on the mass of respiratory particles leaving the N conduit generation. More particularly, the scaling factor can be based on a ratio of the mass of respiratory particles leaving the terminated branch and the mass of respiratory particles leaving the onward branch. For example, the scaling factor can be based on a sum of one and the ratio of the mass of respiratory particles leaving the terminated branch to the mass of respiratory particles leaving the onward branch.

The single conduit model and scale up can provide respiratory particle deposition in all generations downstream of an outlet of the upstream model based on an airflow rate and respiratory particle flow rate of the outlet. The upstream model, however, has multiple outlets. So, to obtain deposition of the respiratory particles over a full lung, multiple single conduit CFD simulations can be performed. The effort involved in performing such multiple single conduit CFD simulations can be reduced by using Reduced Order Model (ROM) techniques. Based on all of the outlets of the upstream model, ranges of parameters like air volume and respiratory particle mass, etc. can be obtained. While using ROMs, it is not necessary to run a single conduit CFD simulation as per the parameters of each outlet of the upstream model. Instead, based on techniques such as Latin Hypercube Sampling (LHS), a smaller number of CFD simulations of the single conduit model can be performed and a ROM can be extracted. This ROM can provide respiratory particle deposition in all generations downstream of an outlet of the upstream generation instantly.

The above summary does not include an exhaustive list of all aspects of the present invention. It is contemplated that the invention includes all devices, systems, and methods that can be practiced from all suitable combinations of the various aspects summarized above, as well as those disclosed in the Detailed Description below and particularly pointed out in the claims filed with the application. Such combinations have particular advantages not specifically recited in the above summary.

## BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings of which:

FIG. 1 is a pictorial view of a lung having several generations, in accordance with an embodiment.

FIG. 2 is a pictorial view of a full lung geometry reduced to a single conduit geometry, in accordance with an embodiment.

FIG. 3 is a flowchart of a method of determining respiratory particle deposition in a lung, in accordance with an embodiment.

FIG. 4 is a pictorial view of single conduit geometry, in accordance with an embodiment.

FIGS. 5A-5B are diagrams of a single conduit model, in accordance with embodiment.

FIG. 6 is a flowchart of a method of generating a reduced order model of respiratory particle deposition in a lung, in accordance with an embodiment.

FIG. 7 is a pictorial view of a reduced order model of respiratory particle deposition in a lung, in accordance with an embodiment.

FIGS. 8-10 depict systems that may be used in conjunction with the embodiments described herein.

## DETAILED DESCRIPTION

Embodiments describe a method and system of modeling respiratory particle deposition in a lung, accurately and efficiently. The system and method can be embodied as software running on a server-oriented system or a standalone computer.

In various embodiments, description is made with reference to the figures. However, certain embodiments may be practiced without one or more of these specific details, or in combination with other known methods and configurations. In the following description, numerous specific details are set forth, such as specific configurations, dimensions, and processes, in order to provide a thorough understanding of the embodiments. In other instances, well-known processes and manufacturing techniques have not been described in particular detail in order to not unnecessarily obscure the description. Reference throughout this specification to "one embodiment," "an embodiment," or the like, means that a particular feature, structure, configuration, or characteristic described is included in at least one embodiment. Thus, the appearance of the phrase "one embodiment," "an embodiment," or the like, in various places throughout this specification are not necessarily referring to the same embodiment. Furthermore, the particular features, structures, configurations, or characteristics may be combined in any suitable manner in one or more embodiments.

The use of relative terms throughout the description may denote a relative position or direction. For example, "distal direction" may indicate a first direction within a lung geometry, e.g., in a downstream direction of air flow during inhalation. Similarly, "proximal direction" may indicate a second direction in an opposite direction from the distal direction, e.g., an upstream direction. Such terms are provided to establish relative frames of reference, however, and are not intended to limit the methods or systems to a specific configuration described in the various embodiments below.

A CFD modeling process for determining respiratory particle deposition in the full lung geometry is provided to improve an accuracy of drug delivery simulation. In an aspect, a method and system of modeling respiratory particle deposition in a lung is provided. The method makes assumptions related to air flow and particle flow that allows higher generations of the lung to be modeled as a single conduit. Simulations of transient air flow and particle flow within conduit generations of the single conduit can be performed to determine particle deposition within the conduit generations. The particle deposition results can then be scaled up to determine total particle deposition in generations of the lung corresponding to the conduit generations. The scaled up drug deposition values account for drug deposition in both a portion of the lung generation that was modeled as part of the single conduit, and in a portion of the lung generation that was not modeled as part of the single conduit. Although based on assumptions, the process provides an estimation of deposition in the lung that is more accurate than 1-dimensional algebraic models and does not require a computationally prohibitive model size. Accordingly, the CFD modeling process described herein can be used to accurately and efficiently determine respiratory particle deposition in a full lung model, including higher generations of the lung.

Referring to FIG. 1, a pictorial view of a lung having several generations is shown in accordance with an embodiment. A human lung 100 has several lung generations 102 of branching. Typically, the lung 100 includes twenty-three generations of branching. Each generation contains branches at a same level in the respiratory tree. Lower generations of the lung 100 encompass a zeroth generation including a trachea, first and second generations including the bronchus, and several generations of bronchioles, e.g., up to a sixth through thirteenth generation. The lower generation branches are relatively large in size, and can be readily scanned to create accurate 3-dimensional geometries for CFD simulation. Generations higher than the sixth through thirteenth generations include terminal bronchioles, respiratory bronchioles, alveolar ducts, and alveoli. The higher generation branches are progressively smaller. For example, alveoli include tissue with small pockets where oxygen and carbon dioxide exchange occurs. The small anatomical structures of the higher generations are not readily scanned to provide a basis for computer simulation.

Referring to FIG. 2, a pictorial view of a full lung geometry reduced to a single conduit geometry is shown in accordance with an embodiment. Despite being complex and multitudinous, the branches of the full lung geometry 200 have a well-defined manner of branching. More particularly, as shown in a full lung geometry 200, a sixth generation branch 202 splits into two seventh generation branches 204, which split into four eighth generation branches 206, which split into eight ninth generation branches 208, and so on. Accordingly, the number of branch outlets at each generation grows as a power of two, e.g., the sixth generation has sixty-four outlets and the twenty-third generation has over eight million outlets.

While the sheer number of branches and outlets can be computationally prohibitive to model, it is contemplated that the full lung geometry 200 may be conceptualized as, and reduced to, an aggregation of single conduit geometries. In the single conduit geometry 210, the sixth generation branch 202 splits into two seventh generation branches 204, which split into two eighth generation branches 206, which split into two ninth generation branches 208, and so on. More particularly, each generation of the single conduit geometry 210 has at most two branches of a corresponding generation of the full lung geometry 200.

As described below, using assumptions related to airflow and particle flow, the single conduit geometry 210 can be used as a basis for representing the more than sixteen million passages of the higher generations, e.g., the seventh generation to the twenty-third generation. By doing so, a CFD modeling process for determining respiratory particle deposition in the full lung geometry 200 can be provided. The process may be advantageous over existing 1-dimensional algebraic models of estimating respiratory particle deposition in the lung because the process using the single conduit geometry 210 can consider 3-dimensional effects.

As described below, the method of determining respiratory particle deposition in higher generations of the lung 100 can use a single conduit model. It will be appreciated, however, that modeling of particle delivery to the lung 100 may also incorporate determining respiratory particle deposition in lower generations of the lung 100. For example, standard CFD modeling of respiratory particle deposition may be performed for the full lung geometry 200 up to a lung generation at which scanning or computational resources become prohibitive. In an embodiment, standard CFD modeling can be performed for the zeroth through some higher generation, e.g., a sixth generation, of the full lung geometry 200. Standard CFD modeling can include one or more of transient air flow and particle flow simulation of respiratory particle deposition in the relatively large branches of the lung 100.

The standard CFD model of the lower generations of the lung 100 can be referred to as an upstream CFD model. The upstream CFD model can produce ranges for several parameters that can be used in the downstream single conduit modeling described below. For example, a range of total air volume exiting an outlet of one of the lower generation branches can be determined. Similarly, a range of a total mass of particles exiting an outlet of one of the lower generation branches can be determined. The ranges can be values of the parameters at different points in time during the inhalation part of a breathing cycle, which is the portion of the breathing cycle that drug is deposited, e.g., when a user breathes in air from an inhaler.

The information about ranges of total air volume or the total mass of particles exiting the lower generation outlet can be provided as input parameters to the CFD modeling process of the higher generation branches of the lung 100. More particularly, the air and particles flowing out of the lower generation branch will enter a higher generation branch.

Accordingly, in addition to determining respiratory particle deposition within the lower generation branches of the lung 100, the upstream model can provide inputs to the CFD modeling process of the higher generation branches of the lung 100.

Referring to FIG. 3, a flowchart of a method of determining respiratory particle deposition in a lung is shown in accordance with an embodiment. The operations of the method shown in FIG. 3 can relate to the illustrations of FIGS. 4-6, and thus, FIGS. 3-6 are described in combination below.

Referring to FIG. 4, a pictorial view of single conduit geometry is shown in accordance with an embodiment. The basis for a CFD simulation of the higher generation branches of the lung 100 can be a single conduit model 402. The single conduit model 402 can be a model of the single conduit geometry 210 described above. At operation 302, the single conduit model 402 is generated. The single conduit model 402 can have conduit generations corresponding to generations of the lung 100. For example, the single conduit model 402 can have a sixth conduit generation 404 corresponding to the sixth generation of the lung 100, a seventh conduit generation 406 corresponding to the seventh generation of the lung 100, an eighth conduit generation 408 corresponding to the eighth generation of the lung 100, and so on. Accordingly, each conduit generation can represent a portion of the corresponding lung generation 102.

The portion of the corresponding lung generation 102 that is represented by each conduit generation can include at most two branches. For example, the sixth conduit generation 404 shown in FIG. 4 includes a single branch corresponding to the sixth generation branch 202. By contrast, the seventh conduit generation 406 and higher conduit generations (eighth, ninth, etc.) include two branches in the illustrated single conduit model 402 corresponding to the seventh generation branch 204. Accordingly, the single conduit model 402 represents the single conduit geometry 210 of the lung 100, which is a portion of the full lung geometry 200. The single conduit model 402 includes interconnected conduit branches providing a continuous airway in the distal direction, and includes one or more bifurcations at which terminated branches 414 extend to outlets that are disconnected from the single conduit.

Each generation of the single conduit model 402 can include a respective inlet and one or more outlets. Taking the seventh conduit generation 406 as an example, the branch can include a conduit inlet 410 receiving air flow from the sixth conduit generation 404. The air flow can move in a distal direction (during inhalation) into branches of the conduit generation. More particularly, the conduit generation can include a bifurcation at which an onward branch 412 and a terminated branch 414 split. The onward branch 412 and the terminated branch 414 receive air flow from the conduit inlet 410. The onward branch 412 can be a branch having an outlet that transfers air into a subsequent conduit generation of the single conduit model 402. More particularly, the onward branch 412 can be continued to create the single conduit. The terminated branch 414 may similarly transfer air distally through an outlet to a subsequent generation of the lung 100. Air flowing through the terminated branch 414, however, flows to an unmodeled portion of the lung 100 (not part of the single conduit model 402), rather than to a modeled portion (part of the single conduit model 402). The terminated branch 414 may therefore, with respect to the single conduit, be considered to be terminated.

The features of the seventh generation described above may also be present in other conduit generations. For example, the sixth conduit generation 404 can include a conduit inlet and a conduit outlet coupled to the conduit inlet 410 of the seventh generation. Similarly, the eighth conduit generation 408 can include a conduit inlet coupled to the conduit outlet 416 of the onward branch 412 of the seventh generation. The eighth conduit can also include a bifurcation at which a respective onward branch and terminated branch emanate and extend to respective outlets. Accordingly, the structure of the conduit generations within the single conduit model 402 is repeatable in that each conduit generation has similar features: a conduit inlet, an onward branch having an onward outlet, and a terminated branch having a terminated outlet.

In an embodiment, the single conduit model 402 is generated based on morphological models of the human lung. For example, a physiologically realistic model of the lung is described in Martonen, et al. 2000 'Human Lung Morphology Models For Particle Deposition Studies', Inhalation Toxicology, Vol. 12 (Supplement 4), pp. 109-121. The single conduit model 402 can be generated based on the geometries described for the lung model.

A number of CFD cases can be run using the single conduit model 402. The method can include determining the number of CFD cases to run. The number can be determined based on the ranges of parameters available from the upstream model. More particularly, a sampling method can be used to determine the number of CFD cases to be run based on the input parameters generated by the upstream model, e.g., the range of total air volume exiting the outlet of one of the lower generation branches and/or the range of a total mass of particles exiting the outlet of one of the lower generation branches. The sampling method can be a Latin Hypercube Sampling method used to generate a near-random sample of parameter values from the ranges for use in the CFD cases. By way of example, and not limitation, the sampling method may generate values for input parameters to be used in ten CFD simulations using different input parameter values within the identified ranges.

Each CFD simulation can be performed on the conduit generations in the higher generations, e.g., seventh to twenty-third generations. More particularly, each CFD simulation can be performed on one or more of the conduit generations of the single conduit model 402. The CFD simulations can use the input parameters from the lower generation simulations, and relevant assumptions and boundary conditions. For example, the CFD simulations can assume laminar air flow through the branches of the conduit generations. Relevant boundary conditions include assigning an air mass flow rate to the branches of the conduit generation. In an embodiment, the boundary conditions assumes that air flow through a conduit generation is half of air flow entering a conduit inlet of a preceding conduit generation. Such assumptions can be useful approximations of actual breathing flow conditions.

The CFD simulation(s) performed on the higher generations of the single conduit model 402 can include transient airflow simulations and particle flow simulations. The transient airflow simulation can use Eulerian methods that apply static conservation of mass, energy, and momentum equations to determine air flow through the single conduit model 402. A transient air flow rate profile can be used at the conduit inlet of the conduit generation being simulated. The transient air flow rate profile can be obtained from the upstream model.

The particle flow simulation can be performed along with the transient air flow simulations. The particle flow simulation can use discrete phase modeling (DPM), which utilizes a dynamic frame of reference, or Lagrangian model. The DPM can determine particle motion and deposition within the single conduit model 402. The DPM can utilize one-way coupled DPM modeling. One-way coupled DPM modeling assumes that air flow affects particle motion, but particle motion does not affect air flow. Based on the particle flow simulation, particle deposition in the conduit generations can be recorded over the full inhalation cycle.

The CFD simulation of respiratory particle delivery to the single conduit model 402 can be used to obtain particle flow and deposition information. For example, the CFD simulation can include operations modeling each of the conduit generations, and the operations can include calculations to obtain particle inflow mass through the conduit inlet of each conduit generation, deposition mass in each conduit generation, and outflow mass for each conduit generation.

At operation 304, a CFD simulation of respiratory particle delivery to the single conduit model is performed. The CFD simulation can include an operation to simulate respiratory particle delivery to a current, or "N," conduit generation of the single conduit model 402. The operation can include a calculation to determine a mass of respiratory particles leaving the N conduit generation. It will be appreciated that the term "current" or "N" conduit generation is used as a relative term, and that the N conduit generation may be any of the conduit generations in the single conduit model 402. In an example, the N conduit generation can be the seventh conduit generation 406 in FIG. 4. The mass of respiratory particles leaving the N conduit generation can include a mass of respiratory particles leaving the onward branch 412 and a mass of respiratory particles leaving the terminated branch 414.

The CFD simulation of respiratory particle delivery to the single conduit model can also include an operation to simulate respiratory particle delivery to a next, "N+1," conduit generation of the single conduit model 402. It will be appreciated that the term "next" or "N+1" conduit generation is used as a relative term, and that the N+1 conduit generation may be the conduit generation subsequent to the N conduit generation. In the example, the N+1 conduit generation is the eighth conduit generation 408 in FIG. 4. More particularly, the N+1 conduit generation can be a conduit generation directly adjacent to and connected to the N conduit generation of operation 304. More particularly, the onward branch 412 of the N conduit generation simulated at operation 304 may be connected to a conduit inlet of the N+1 conduit generation being calculated at operation 306. The operation of CFD simulation can include a calculation to determine a mass of respiratory particles deposited in the N+1 conduit generation.

Although the CFD simulation at operation 304 can determine the particle inflow mass, deposition, and outflow mass for the conduit generations, and not merely the highlighted determinations for the N conduit generation and the N+1 conduit generation described above, the mass of respiratory particles leaving the N conduit generation and the mass of respiratory particles deposited in the N+1 conduit generation are of particular importance to the next stage of the CFD process: scaling up the single conduit model 402 to a full model of the lung 100. The scaling up process can include post-processing that uses the results of the CFD simulation on the single conduit model to determine total particle deposition within one or more generations of the lung.

Referring to FIG. 5A, a diagram of a single conduit model is shown in accordance with an embodiment. Scaling up the single conduit results can be used to determine a total deposition of respiratory particles in the higher generations of the lung 100. More particularly, the particle deposition of unmodeled conduits can be determined from the single conduit CFD model. If particle flow divided symmetrically through branches in the distal direction, then scaling up particle deposition calculated within conduit generations to determine total deposition in a corresponding generation of the lung 100 would be a relatively straightforward process. More particularly, the deposition within the modeled single conduit generation could be multiplied by the number of unmodeled conduit generations in the corresponding lung generation to arrive at the total deposition. For example, as shown in FIG. 5A, the eighth generation of the lung 100 includes the eighth conduit generation 408 and a corresponding phantom conduit generation 502, which is an unmodeled portion of the eighth generation of the lung 100. The phantom conduit generation 502 connects to the terminated branch 414 of the seventh conduit generation 406 and, thus, if particle flow divided symmetrically between the branches of the seventh generation, then the total particle deposition in the eighth generation could be determined by multiplying the particle deposition in the eighth conduit generation 408 by two. In reality, however, particle flow does not divide evenly and thus methodologies that adjust for asymmetric particle flow within the lung branches may be needed to accurately scale up the single conduit results to determine the total deposition of the respiratory particles in the higher generations of the lung 100.

As described above, the single conduit CFD model can provide the mass of respiratory particles deposited in the sixth conduit generation 404 and the seventh conduit generation 406, by way of example. The branches of the sixth conduit generation 404 and the seventh conduit generation 406 can be part of the single conduit model 402, and thus, can be modeled portions of the lung 100. By contrast, with respect to the eighth conduit generation 408, a deposited particle mass on a wall of the eighth conduit generation 408 can be known, however, particle deposition on an unmodeled portion (the phantom conduit generation 502) of the eighth generation may be unknown. Using stoichiometry, deposition in phantom conduit generations 502 (the unmodeled branches) of a lung generation can be estimated based on a ratio of mass flow rate leaving the onward branch 412 and terminated branch 414 of a preceding conduit generation. Such a ratio can account for asymmetric particle flow.

At operation 306, determining a total mass of respiratory particles deposited in the generation of the lung 100 corresponding to the N+1 conduit generation (e.g., the eighth conduit generation 408) is based on the mass of respiratory particles deposited in the N+1 conduit generation and a scaling factor for the N+1 conduit generation. The scaling factor can be based on the mass of respiratory particles leaving the N conduit generation (the conduit generation preceding the N+1 conduit generation or, in this case, the seventh conduit generation 406). More particularly, the total deposition in the N+1 conduit generation is calculated by multiplying the N+1 generation mass deposition obtained from the model with a multiplication factor, and the multiplication factor is based on particle flow leaving the N conduit generation.

To determine the scaling factor, particle mass flowing through each of the conduit generations can first be determined. In the example, the particle mass entering the sixth conduit generation 404 (which may be termed a previous conduit generation) may be termed $mp6_i$. A value of $mp6_i$ can be input from the upstream model leading to the previous conduit generation. Furthermore, the deposited particle mass on the sixth conduit generation 404 wall may be termed $mp6_d$. A value of $mp6_d$ may be determined from an operation of the CFD simulation performed on the sixth conduit generation 404.

With respect to the seventh conduit generation 406 (or the so-called N conduit generation), a deposited particle mass on the seventh conduit generation 406 wall may be termed $mp7_d$ and determined from an operation of the CFD simulation performed on the seventh conduit generation 406. Particle mass leaving the N conduit generation through the terminated branch 414 may be termed $mp7_e$.

With known values for the deposited particle mass of the seventh conduit generation 406 and the particle mass exiting the seventh conduit generation 406 through the terminated branch 414, the particle mass flowing onward through the onward branch 412 to the eighth conduit generation 408 can be determined. A mass balance can be used to determine the onward flow of each conduit generation. For example, the onward flow is equal to what goes into the conduit generation minus what is deposited on the wall of the conduit generation minus what leaves the terminated branch of the conduit generation. Accordingly, the onward particle mass from the seventh conduit generation 406 is defined by the equation: $mp7_o = mp6_i - mp6d - mp7d - mp7e$. The equation can be solved to determine $mp7_o$, which may or may not be equal to $mp7_e$.

The particle mass deposited on a wall of the eighth conduit generation 408 can be determined from the CFD simulation. The deposited particle mass in the phantom conduit generation 502, however, is unknown. The particle mass flowing into the eighth conduit generation 408 and the phantom conduit generation 502 from the seventh conduit generation 406 is known, however, and stoichiometry can be used to determine the deposition in the phantom conduit generation 502 and, more particularly, the total deposition in the eighth generation of the lung 100.

It will be appreciated that the onward particle mass entering the eighth conduit generation 408 from the onward branch 412 of the seventh conduit generation 406, $mp7_o$, correlates to a particle mass deposited on a wall of the eighth conduit generation 408, $mp8_{d1}$. Similarly, the particle mass leaving the seventh conduit generation 406, $mp7_e$ correlates to a particle mass deposited on a wall of the phantom conduit generation 502, $mp8_{d2}$. Based on stoichiometry, it follows that $mp8_{d2}$ relates to $mp8_{d1}$ through a ratio of $mp7_e$ and $mp7_o$. More particularly, the deposited particle mass can be determined by the equation: $mp8_{d2} = \left(\frac{mp7_e}{mp7_o}\right) * \ mp8_{d1}$. This can be extended to an equation describing a total deposition in the eighth generation of the lung 100:

$$Total\ Deposition = \ mp8_{d1} + mp8_{d2} = mp8_{d1} + \left(\frac{mp7_e}{mp7_o}\right) * mp8_{d1} = mp8_{d1} * \ (1 + \frac{mp7_e}{mp7_o})$$

. Using this equation, the scaling factor, MF8, for the eighth generation of the lung 100 can be defined as $1 + \frac{mp7_e}{mp7_o}$. The scaling factor can be used to scale up the deposited mass in the eighth conduit generation 408 by multiplying the deposited particle mass on the eighth conduit generation wall by MF8.

Based on the above description, it will be appreciated that the scaling factor used to scale up deposition in an N+1 generation can be based on a ratio of the mass of respiratory particles leaving the terminated branch 414 and the mass of respiratory particles leaving the onward branch 412 of the N (previous) generation. The scaling factor can be determined based on the ratio. To generalize, the scaling factor can be based on a sum of one and the ratio of the mass of respiratory particles leaving the terminated branch 414 to the mass of respiratory particles leaving the onward branch 412. The total deposition in the modeled and unmodeled portions of the N+1 generation of the lung 100 can then be calculated by multiplying the particle deposition in the N+1 conduit generation determined using the CFD model by the scaling factor. For example, the total deposited in the ninth generation is calculated by multiplying the ninth generation mass deposition obtained from the single conduit model with a multiplication factor calculated using the ratio of the previous generation (the eighth generation) mass flow rate from the onward and terminated branches of the previous generation. Similarly, the total deposition in any generation can be calculated as the deposition in the generation being analyzed times the scaling factors of every preceding generation. For example, a total deposition in the tenth generation is obtained by multiplying the tenth generation mass deposition obtained from the single conduit model with the scaling factor for the eighth, ninth, and the tenth generation calculated using the ratio of the previous generation mass flow rate from the onward and terminated branches of the previous generation. Accordingly, a total deposition in any generation can be calculated using the single conduit modeled mass deposition for the generation and an accumulated multiplication factor.

Referring to FIG. 5B, a diagram of a single conduit model is shown in accordance with an embodiment. To further describe the process of scaling up single conduit results in subsequent generations, a method of scaling up a ninth conduit generation 510 will now be described. Like the eighth generation, for the ninth generation, a multiplication factor can be derived from the ratio of onward and terminated mass flow in the eighth conduit generation 408. The scaling factor (also termed a multiplication factor) for the ninth generation can be defined by the equation: $MF9 = 1 + \frac{mp8_e}{mp8_o}$. The scaling factor, which like MF8 is based on a sum of one and the ratio of the mass of respiratory particles leaving the terminated branch of the preceding conduit generation to the mass of respiratory particles leaving the onward branch of the preceding conduit generation, can be used to scale up the mass deposition in the ninth conduit generation 510. So, the total deposition in the ninth conduit generation 510 downstream of the eighth conduit generation 408 equals: $mp9_{d1} * MF9$. In the case of the ninth generation, however, rather than having one phantom conduit generation 502 connected to the terminated branch of the preceding conduit generations, there are three phantom conduit generations 502. That is, one phantom conduit generation 502 connects to the terminated branch 414 of the eighth conduit generation 408, and two phantom conduit generations 502 connect to the phantom conduit generation 502 of the eighth conduit generation 408.

The scaling factor MF9 accounts for the phantom conduit generation 502 connected to the terminated branch 414 of the eighth conduit generation 408, but not for the other two phantom conduit generations 502. To account for the other two phantom conduit generations 502, stoichiometry may be used. Similar to the description above, it will be appreciated that the particle mass leaving the onward branch of the seventh conduit generation 406, $mp7_o$, correlates to a particle mass deposited on a wall of the ninth conduit generation 510 and, specifically, results in the deposition on the wall defined by the equation $mp9_{d1} * MF9$. Similarly, the particle mass leaving the terminated branch of the seventh conduit generation 406, $mp7_e$ correlates to a particle mass deposited on a wall of the phantom conduit generations 502 that connect to the phantom conduit generation 502 of the eighth generation, $mp9_{d3}$. Based on stoichiometry, it follows that $mp9_{d3}$ relates to $mp9_{d1}$ through a ratio of $mp7_e$ and $mp7_o$. More particularly, $mp9_{d3}$ can be determined by the equation: $mp9_{d3} = \left(\frac{mp7_e}{mp7_o}\right) * mp9_{d1} * MF9$. This can be extended to an equation describing a total deposition in the ninth generation of the lung: $Total\ Deposition = mp9 * MF9 + \left(\frac{mp7_e}{mp7_o}\right) * mp9_{d1} * MF9$. The equation simplifies to: $mp9_{d1} * MF9 * \left(1 + \frac{mp7_e}{mp7_o}\right)$. As described above, 1 + $\frac{mp7_e}{mp7_o}$ can be the scaling factor for the eighth conduit generation 408. Thus, the ninth conduit generation 510 can be scaled up by multiplying the deposited particle mass on the ninth conduit generation 510 wall by the scaling factor of the ninth generation and all of the preceding generations, e.g., MF8.

The process of scaling up the conduit generation for subsequent generations can follow the same paradigm as described above. More particularly, for the tenth generation, the total deposition can be determined according to the equation: $mp10_{d1} * MF10 * MF9 * MF8$, where $mp10_{d1}$ is the deposition on the tenth conduit generation wall as determined by the single conduit CFD simulation, and MF10 equals $1 + (\frac{mp9_e}{mp9_o})$. For the eleventh generation, the total deposition can be determined according to the equation: $mp11_{d1} * MF11 * MF10 * MF9 * MF8$, where $mp11_{d1}$ is the deposition on the eleventh conduit generation wall as determined by the single conduit CFD simulation, and MF11 equals $1 + (\frac{mp10_e}{mp10_o})$. Subsequent generations, e.g., up to the twenty-third generation, can have their total deposition masses similarly calculated.

Referring to FIG. 6, a flowchart of a method of generating a reduced order model of respiratory particle deposition in a lung is shown in accordance with an embodiment. As described above, several CFD simulations can be performed on the single conduit model based on parameter values determined by a sampling method, e.g., a Latin Hypercube Sampling design-of-experiment. More particularly, rather than model every possible set of input parameters, a predetermined group of input parameters may be modeled and then a reduced order model (ROM) can be generated to provide a response surface that describes all possible input parameter combinations and particle deposition.

At operation 602, additional CFD simulations of respiratory particle delivery to the single conduit model are performed. The additional CFD simulations can be used to determine total masses of respiratory particles deposited in a generation of the lung 100 corresponding to the N+1 conduit generation for a range of an input parameter. The range of the input parameter can be output from the upstream model, as described above. For example, the input parameter can be one or more of total particle mass entering the N conduit generation or total air volume entering the N conduit generation. Furthermore, the number of additional CFD simulations can be based on the range of the input parameter, as described above. At operation 604, a ROM including total masses of respiratory particles deposited in the generation of the lung 100 is generated.

Referring to FIG. 7, a pictorial view of a reduced order model of respiratory particle deposition in a lung is shown in accordance with an embodiment. The total deposition data from the CFD runs can be used to build a ROM 702. The ROM 702 is a smart interpolation of a few CFD runs. More particularly, the ROM 702 can be a scalar ROM. That is, for given values of input parameters, the ROM 702 can provide drug deposition in the higher generations of the lung 100.

The ROM 702 can predict an output from received one or more inputs based on a response surface 704 that is generated from the different runs of the scaling up method. The response surface 704 is illustrated as a plot with a first input parameter 706, e.g., total air volume entering the single conduit model 402, a second input parameter 708, e.g., total particle mass entering the single conduit model 402, and an output parameter 710, e.g., total respiratory particle deposition in one or more of the lung generations 102. More particularly, there can be a separate response surface 704 for every generation modeled, and for any given values of the input parameters, the corresponding output parameter 710 for the modeled generation can be determined.

The input parameters 706, 708 can have ranges of input parameters that come from the upstream model. More particularly, the input parameters can correspond to characteristics of air flow or particles entering the N conduit generation (or, conversely, leaving an N-1 conduit generation that has an outlet connected to an inlet of the N conduit generation). Such characteristics can include breathing profiles of air flow within the lower generation conduits that include the N-1 conduit generation, particle mass and/or size distribution of particles leaving the N-1 conduit generation, etc. Such input parameters can be output from simulations of a single upstream lung model or several upstream lung models.

In an embodiment, the ROM 702 is used to calculate deposition downstream of all outlets of the upstream model. More particularly, the ROM 702 can be used to calculate the deposition in the full lung 100. For example, the ROM 702, which is created from multiple CFD simulations of the single conduit model, can provide values of particle deposition for any combination of input parameters. The input parameters can include air flow volume and particle mass obtained from the upstream model, and input of the parameter values can cause an output of a corresponding total particle deposition for a conduit generation, a generation of the lung 100 corresponding to the conduit generation, or the full lung 100 including all lower and higher generation branches.

The ROM 702 can be applied to obtain results of drug deposition simulation to a lung with a set of input parameters, e.g., a set of boundary conditions. More particularly, after the ROM is built, a set of one or more values of input parameter(s) can be determined. The determination of the input parameter(s) can include performing a CFD simulation of respiratory particle delivery to the upstream lung model. For example, the CFD simulation can simulate respiratory particle delivery to the N-1 conduit generation of the upstream lung model immediately preceding the N conduit generation of the higher conduit generations. The result of the simulation can be values of input parameters that are plotted against a total mass of respiratory particles deposited in the lung. For example, the input parameter can be total particle mass leaving the N-1 conduit generation and/or total air volume leaving the N-1 conduit generation. The ROM may then be applied using the input parameter(s) to determine a total mass of respiratory particles deposited in the lung. The ROM 702, which is created using the CFD modeling process described above, can therefore provide an efficient manner of quickly determining the efficacy of particle deposition based on the modeled input parameters.

The methods and systems described herein may be implemented using any suitable processing system with any suitable combination of hardware, software and/or firmware, such as described below with reference to the non-limiting examples of FIGS. 9-11. The methods described above may therefore be regarded as computer-implemented methods.

Referring to FIG. 8, a system that may be used in conjunction with the embodiments described herein is shown. FIG. 8 depicts at 800 a computer-implemented environment wherein users 804 can interact with a system 806 hosted on one or more servers 808 through a network 810. The system 806 contains software operations or routines. The users 804 can interact with the system 806 through a number of ways, such as over one or more networks 810. One or more servers 808 accessible through the network(s) 810 can host system 806. The servers 808 can have access to data stores 812. The one or more data stores 812 may contain first data 814 as well as second data 816. It should be understood that the system 806 could also be provided on a standalone computer for access by a user.

Referring to FIG. 9, a system that may be used in conjunction with the embodiments described herein is shown. FIG. 9 depicts an exemplary system 900 that includes a standalone computer architecture where a processing system 902 (e.g., one or more computer processors) includes a system 904 being executed on it. The processing system 902 has access to a non-transitory computer-readable memory 906 in addition to one or more data stores 908. The one or more data stores 908 may contain first data 910 as well as second data 912. The data can include instructions executable by the processing system 902 to cause the system 900 to perform the methods described above.

Referring to FIG. 10, a system that may be used in conjunction with the embodiments described herein is shown. FIG. 10 shows a block diagram of exemplary hardware for a standalone computer architecture 1000, such as the architecture depicted in FIG. 9, that may be used to contain and/or implement the program instructions of system embodiments of the present invention. A bus 1002 may serve as the information highway interconnecting the other illustrated components of the hardware. A processing system 1004 labeled CPU (central processing unit) (e.g., one or more computer processors), may perform calculations and logic operations required to execute a program. A non-transitory computer-readable storage medium, such as read only memory (ROM) 1006 and random access memory (RAM) 1008, may be in communication with the processing system 1004 and may contain one or more programming instructions. Optionally, program instructions may be stored on a non-transitory computer-readable storage medium such as a magnetic disk, optical disk, recordable memory device, flash memory, or other physical storage medium. Computer instructions may also be communicated via a communications signal, or a modulated carrier wave, e.g., such that the instructions may then be stored on a non-transitory computer-readable storage medium.

A disk controller 1010 interfaces one or more optional disk drives to the system bus 1002. These disk drives may be external or internal floppy disk drives such as 1012, external or internal CD-ROM, CD-R, CD-RW or DVD drives such as 1014, or external or internal hard drives 1016. As indicated previously, these various disk drives and disk controllers are optional devices.

Each of the element managers, real-time data buffer, conveyors, file input processor, database index shared access memory loader, reference data buffer and data managers may include a software application stored in one or more of the disk drives connected to the disk controller 1010, the ROM 1014 and/or the RAM 1016. Preferably, the processor 1004 may access each component as required.

A display interface 1020 may permit information from the bus 1002 to be displayed on a display 1022 in audio, graphic, or alphanumeric format. Communication with external devices may optionally occur using various communication ports 1024.

In addition to the standard computer-type components, the hardware may also include data input devices. The data input devices can transfer information to the bus 1002 through an interface 1028. The data input device can include a keyboard 1030, or other input device, such as a microphone 1032, remote control, pointer, mouse, touchscreen and/or joystick.

This written description describes exemplary embodiments of the invention, but other variations fall within the scope of the disclosure. For example, the systems and methods may include and utilize data signals conveyed via networks (e.g., local area network, wide area network, internet, combinations thereof, etc.), fiber optic medium, carrier waves, wireless networks, etc. for communication with one or more data processing devices. The data signals can carry any or all of the data disclosed herein that is provided to or from a device.

The methods and systems described herein may be implemented on many different types of processing devices by program code comprising program instructions that are executable by the device processing system. The software program instructions may include source code, object code, machine code, or any other stored data that is operable to cause a processing system to perform the methods and operations described herein. Any suitable computer languages may be used such as C, C++, Java, etc., as will be appreciated by those skilled in the art. Other implementations may also be used, however, such as firmware or even appropriately designed hardware configured to carry out the methods and systems described herein.

The systems' and methods' data (e.g., associations, mappings, data input, data output, intermediate data results, final data results, etc.) may be stored and implemented in one or more different types of computer-implemented data stores, such as different types of storage devices and programming constructs (e.g., RAM, ROM, Flash memory, flat files, databases, programming data structures, programming variables, IF-THEN (or similar type) statement constructs, etc.). It is noted that data structures describe formats for use in organizing and storing data in databases, programs, memory, or other non-transitory computer-readable media for use by a computer program.

The computer components, software modules, functions, data stores and data structures described herein may be connected directly or indirectly to each other in order to allow the flow of data needed for their operations. It is also noted that a module or processor includes but is not limited to a unit of code that performs a software operation, and can be implemented for example as a subroutine unit of code, or as a software function unit of code, or as an object (as in an object-oriented paradigm), or as an applet, or in a computer script language, or as another type of computer code. The software components and/or functionality may be located on a single computer or distributed across multiple computers depending upon the situation at hand.

It should be understood that as used in the description herein and throughout the claims that follow, the meaning of "a," "an," and "the" includes plural reference unless the context clearly dictates otherwise. Also, as used in the description herein and throughout the claims that follow, the meaning of "in" includes "in" and "on" unless the context clearly dictates otherwise. Finally, as used in the description herein and throughout the claims that follow, the meanings of "and" and "or" include both the conjunctive and disjunctive and may be used interchangeably unless the context expressly dictates otherwise; the phrase "exclusive or" may be used to indicate situation where only the disjunctive meaning may apply.

The preceding detailed descriptions are presented in terms of algorithms and symbolic representations of operations on data bits within a device memory. These algorithmic descriptions and representations are the tools used by those skilled in the data processing arts to most effectively convey the substance of their work to others skilled in the art. An algorithm is here, and generally, conceived to be a self-consistent sequence of operations leading to a desired result. The operations are those requiring physical manipulations of physical quantities. Usually, though not necessarily, these quantities take the form of electrical or magnetic signals capable of being stored, transferred, combined, compared, and otherwise manipulated. It has proven convenient at times, principally for reasons of common usage, to refer to these signals as bits, values, elements, symbols, characters, terms, numbers, or the like.

It should be kept in mind, however, that all of these and similar terms are to be associated with the appropriate physical quantities and are merely convenient labels applied to these quantities. Unless specifically stated otherwise as apparent from the above discussion, it is appreciated that throughout the description, discussions utilizing terms such as "receiving," "determining," "sending," "terminating," "waiting," "changing," or the like, refer to the action and processes of a device, or similar electronic computing device, that manipulates and transforms data represented as physical (electronic) quantities within the device's registers and memories into other data similarly represented as physical quantities within the device memories or registers or other such information storage, transmission or display devices.

The processes and displays presented herein are not inherently related to any particular device or other apparatus. Various general-purpose systems may be used with programs in accordance with the teachings herein, or it may prove convenient to construct a more specialized apparatus to perform the operations described. The required structure for a variety of these systems will be evident from the description below. In addition, the disclosure is not described with reference to any particular programming language. It will be appreciated that a variety of programming languages may be used to implement the teachings of the disclosure as described herein.

In the foregoing specification, the invention has been described with reference to specific exemplary embodiments thereof. It will be evident that various modifications may be made thereto without departing from the broader spirit and scope of the invention as set forth in the following claims. The specification and drawings are, accordingly, to be regarded in an illustrative sense rather than a restrictive sense.

## Claims

**1.** A non-transitory computer-readable medium storing instructions executable by one or more processors of a system to cause the system to perform a method, comprising:

generating a single conduit model having conduit generations representing portions of corresponding generations of a lung, wherein the portions include at most two branches of the corresponding generations of the lung;
performing a computational fluid dynamics (CFD) simulation of respiratory particle delivery to the single conduit model to determine a mass of respiratory particles leaving an N conduit generation and a mass of respiratory particles deposited in an N+1 conduit generation; and
determining a total mass of respiratory particles deposited in a generation of the lung corresponding to the N+1 conduit generation based on the mass of respiratory particles deposited in the N+1 conduit generation and a scaling factor for the N+1 conduit generation, wherein the scaling factor is based on the mass of respiratory particles leaving the N conduit generation.

2. The non-transitory computer-readable medium of claim 1, wherein the N conduit generation includes an onward branch and a terminated branch, and wherein the mass of respiratory particles leaving the N conduit generation includes a mass of respiratory particles leaving the onward branch and a mass of respiratory particles leaving the terminated branch.

3. The non-transitory computer-readable medium of claim 2, wherein the scaling factor is based on a ratio of the mass of respiratory particles leaving the terminated branch and the mass of respiratory particles leaving the onward branch.

4. The non-transitory computer-readable medium of claim 3, wherein the scaling factor is based on a sum of one and the ratio of the mass of respiratory particles leaving the terminated branch to the mass of respiratory particles leaving the onward branch.

5. The non-transitory computer-readable medium of claim 1 further comprising:

performing additional CFD simulations of respiratory particle delivery to the single conduit model to determine total masses of respiratory particles deposited in the generation of the lung for a range of an input parameter; and
generating a reduced order model (ROM) to predict the total masses of respiratory particles deposited in the generation of the lung.

6. The non-transitory computer-readable medium of claim 5, wherein a number of the additional CFD simulations is based on the range of the input parameter.

7. The non-transitory computer-readable medium of claim 5, wherein the input parameter is one or more of total particle mass entering the N conduit generation or total air volume entering the N conduit generation.

8. A computer-implemented method, comprising:

receiving, by a memory of a system, a single conduit model having conduit generations representing portions of corresponding generations of a lung, wherein the portions include at most two branches of the corresponding generations of the lung;
performing, by one or more processors of the system, a computational fluid dynamics (CFD) simulation of respiratory particle delivery to the single conduit model to determine a mass of respiratory particles leaving an N conduit generation and a mass of respiratory particles deposited in the N+1 conduit generation; and
determining, by the one or more processors, a total mass of respiratory particles deposited in a generation of the lung corresponding to the N+1 conduit generation based on the mass of respiratory particles deposited in the N+1 conduit generation and a scaling factor for the N+1 conduit generation, wherein the scaling factor is based on the mass of respiratory particles leaving the N conduit generation.

9. The computer-implemented method of claim 8, wherein the N conduit generation includes an onward branch and a terminated branch, and wherein the mass of respiratory particles leaving the N conduit generation includes a mass of respiratory particles leaving the onward branch and a mass of respiratory particles leaving the terminated branch.

10. The computer-implemented method of claim 9, wherein the scaling factor is based on a ratio of the mass of respiratory particles leaving the terminated branch and the mass of respiratory particles leaving the onward branch.

11. The computer-implemented method of claim 10, wherein the scaling factor is based on a sum of one and the ratio of the mass of respiratory particles leaving the terminated branch to the mass of respiratory particles leaving the onward branch.

12. The computer-implemented method of claim 8 further comprising:

performing, by the one or more processors, additional CFD simulations of respiratory particle delivery to the single conduit model to determine total masses of respiratory particles deposited in the generation of the lung for a range of an input parameter; and
generating, by the one or more processors, a reduced order model (ROM) to predict the total masses of respiratory particles deposited in the generation of the lung.

**13.** A system, comprising:

a memory to receive a single conduit model having conduit generations representing portions of corresponding generations of a lung, wherein the portions include at most two branches of the corresponding generations of the lung; and
one or more processors configured to:

perform a computational fluid dynamics (CFD) simulation of respiratory particle delivery to the single conduit model to determine a mass of respiratory particles leaving an N conduit generation and a mass of respiratory particles deposited in the N+1 conduit generation, and
determine a total mass of respiratory particles deposited in a generation of the lung corresponding to the N+1 conduit generation based on the mass of respiratory particles deposited in the N+1 conduit generation and a scaling factor for the N+1 conduit generation, wherein the scaling factor is based on the mass of respiratory particles leaving the N conduit generation.

**14.** The system of claim 13, wherein the N conduit generation includes an onward branch and a terminated branch, and wherein the mass of respiratory particles leaving the N conduit generation includes a mass of respiratory particles leaving the onward branch and a mass of respiratory particles leaving the terminated branch.

**15.** The system of claim 14, wherein the scaling factor is based on a ratio of the mass of respiratory particles leaving the terminated branch and the mass of respiratory particles leaving the onward branch.

**16.** The system of claim 15, wherein the scaling factor is based on a sum of one and the ratio of the mass of respiratory particles leaving the terminated branch to the mass of respiratory particles leaving the onward branch.

**17.** The system of claim 13, wherein the one or more processors are configured to:

perform additional CFD simulations of respiratory particle delivery to the single conduit model to determine total masses of respiratory particles deposited in the generation of the lung for a range of an input parameter; and
generate a reduced order model (ROM) to predict the total masses of respiratory particles deposited in the generation of the lung.

**18.** A non-transitory computer-readable medium storing instructions executable by one or more processors of a system to cause the system to perform a method, comprising:

perform a computational fluid dynamics (CFD) simulation of respiratory particle delivery to an N-1 conduit generation to determine an input parameter; and
apply a reduced order model (ROM) using the input parameter to determine a total mass of respiratory particles deposited in a lung.

**19.** The non-transitory computer-readable medium of claim 18, wherein the ROM is a scalar ROM having the input parameter plotted against the total mass of respiratory particles deposited in the lung.

**20.** The non-transitory computer-readable medium of claim 18, wherein the input parameter is one or more of total particle mass leaving the N-1 conduit generation or total air volume leaving the N-1 conduit generation.

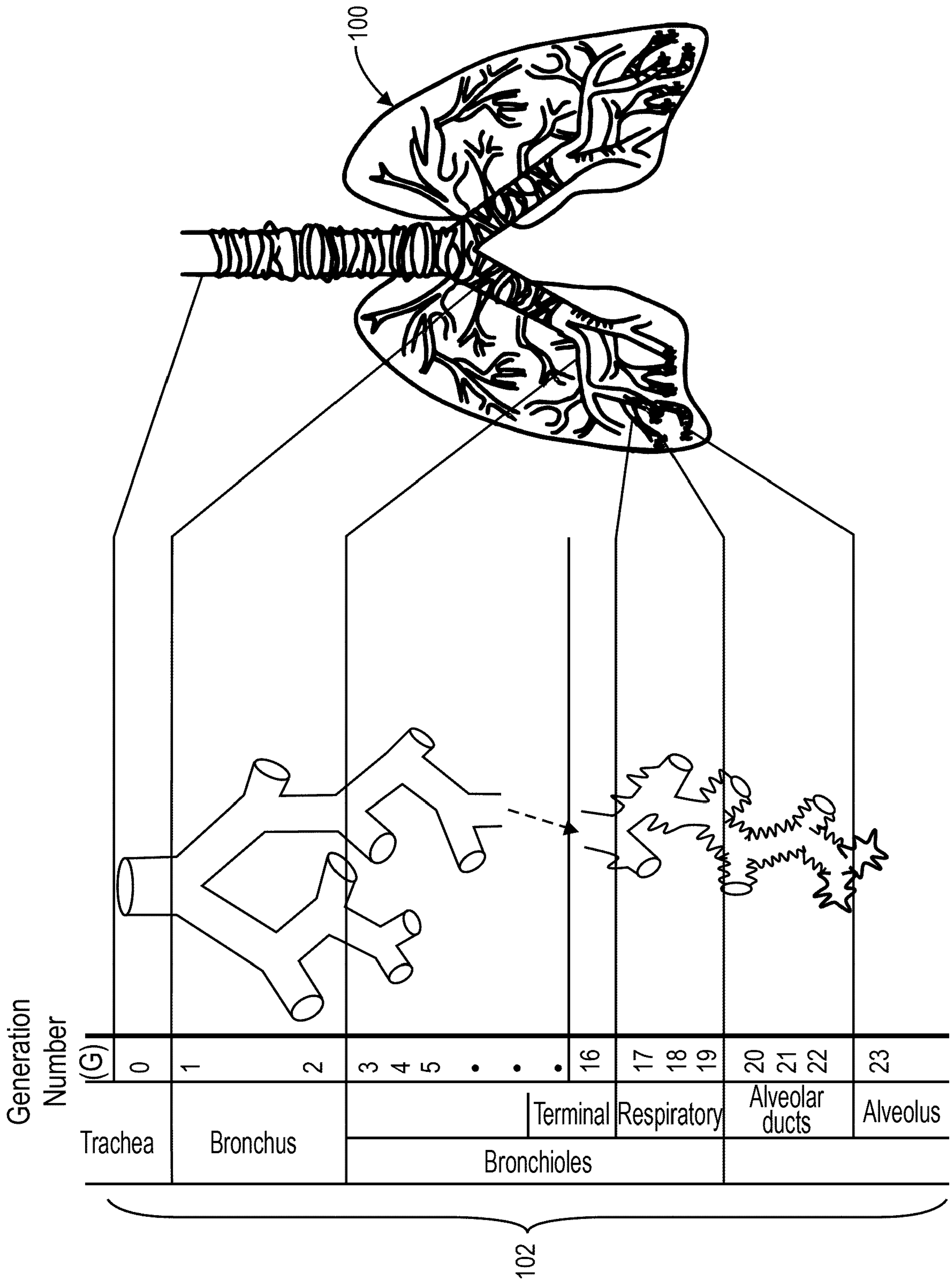
100
Generation Number (G)
0
1
2
3 4 5 • • •
16
17 18 19
20 21 22
23
Trachea
Bronchus
Terminal
Respiratory
Alveolar ducts
Alveolus
Bronchioles
102

FIG. 1

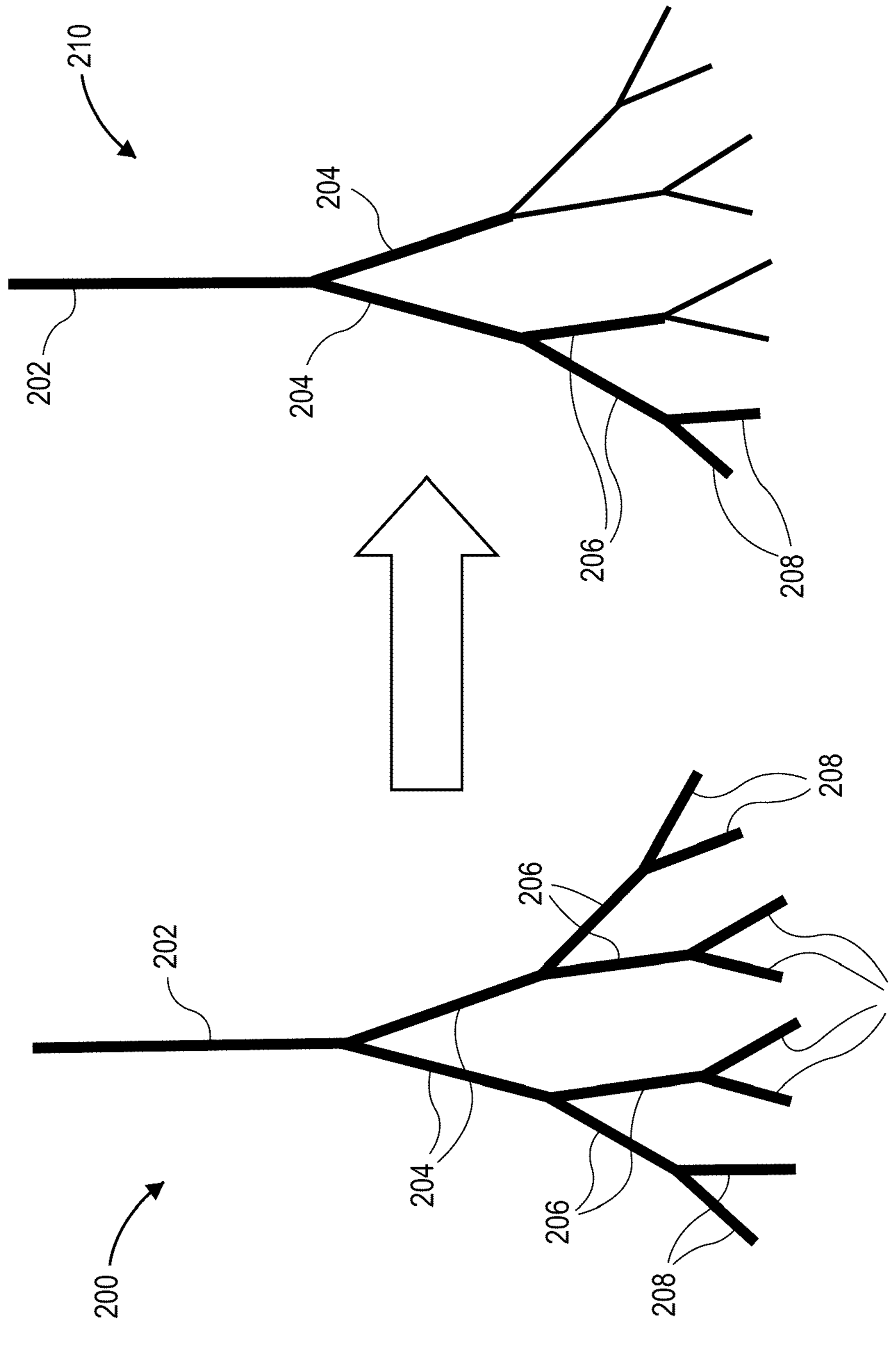


FIG. 2

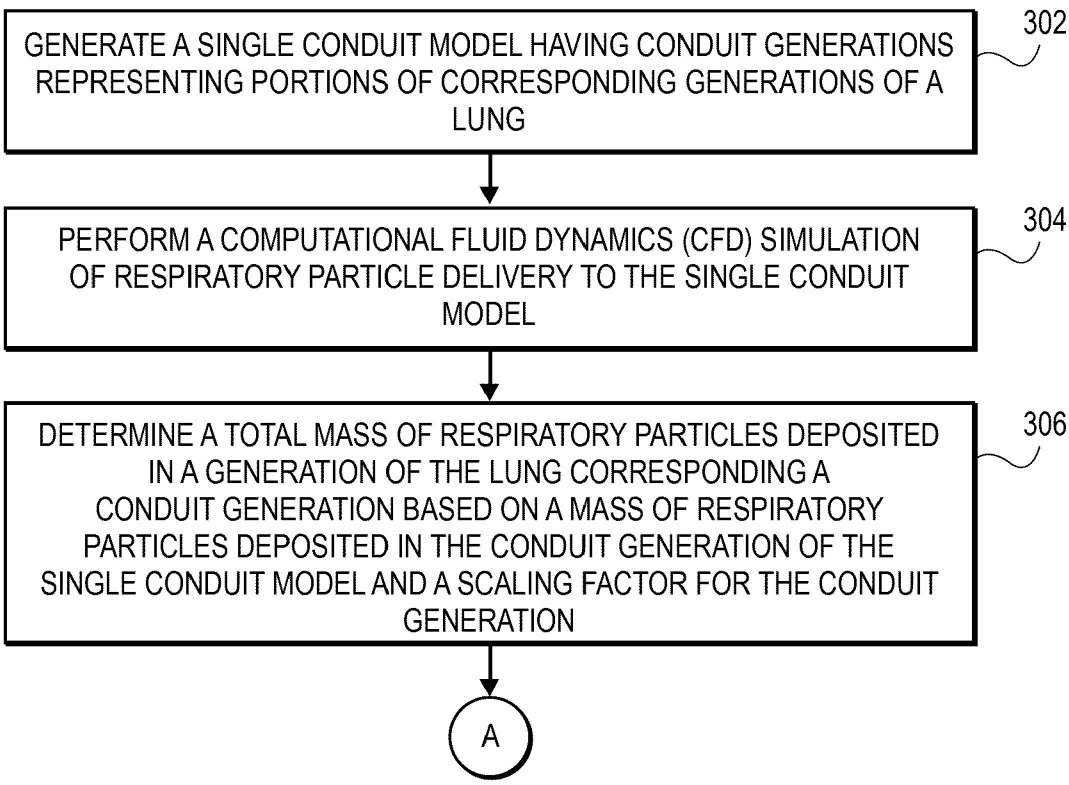
GENERATE A SINGLE CONDUIT MODEL HAVING CONDUIT GENERATIONS REPRESENTING PORTIONS OF CORRESPONDING GENERATIONS OF A LUNG
302
PERFORM A COMPUTATIONAL FLUID DYNAMICS (CFD) SIMULATION OF RESPIRATORY PARTICLE DELIVERY TO THE SINGLE CONDUIT MODEL
304
DETERMINE A TOTAL MASS OF RESPIRATORY PARTICLES DEPOSITED IN A GENERATION OF THE LUNG CORRESPONDING A CONDUIT GENERATION BASED ON A MASS OF RESPIRATORY PARTICLES DEPOSITED IN THE CONDUIT GENERATION OF THE SINGLE CONDUIT MODEL AND A SCALING FACTOR FOR THE CONDUIT GENERATION
306
A

FIG. 3

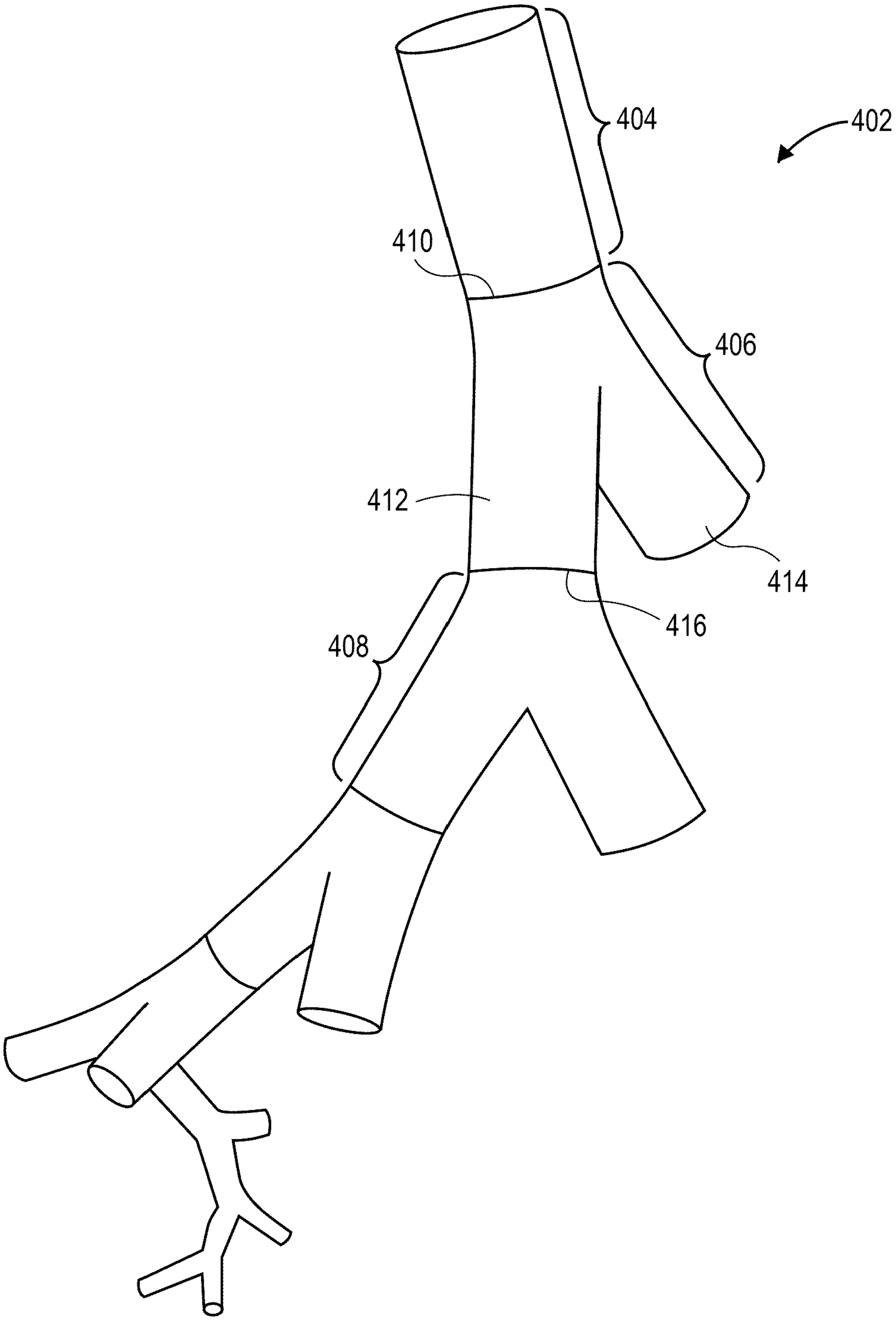


FIG. 4

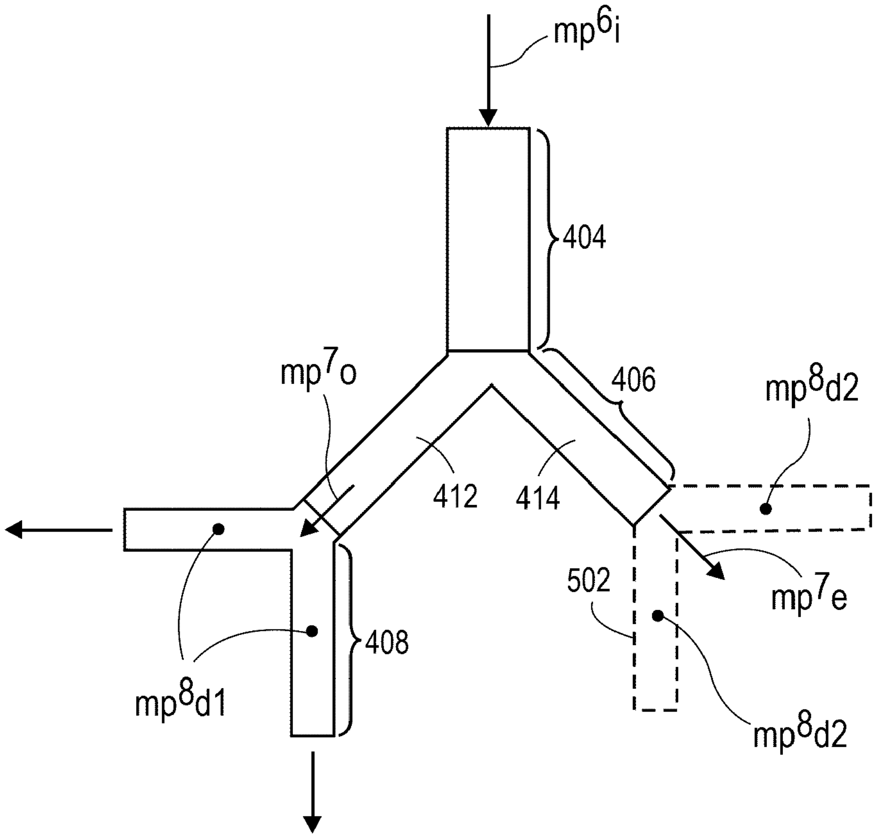


FIG. 5A

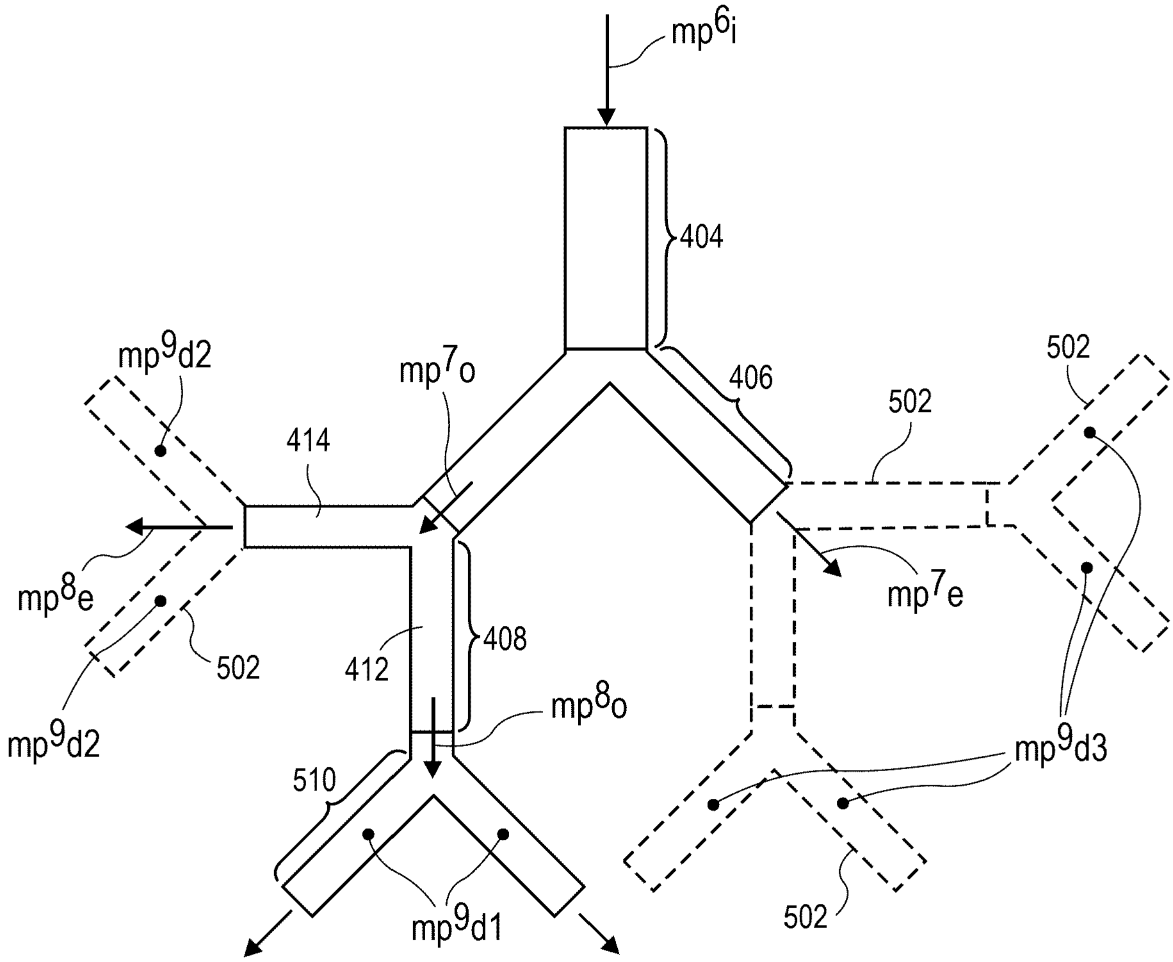
mp6i
404
406
502
502
mp9d2
mp7o
414
mp8e
502
mp9d2
412
408
mp8o
mp7e
mp9d3
510
mp9d1
502

FIG. 5B

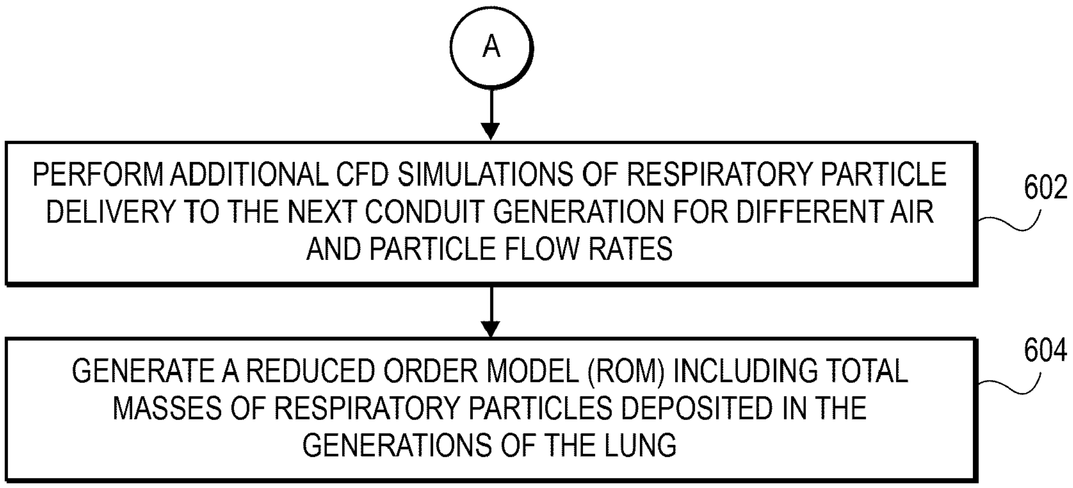
A
PERFORM ADDITIONAL CFD SIMULATIONS OF RESPIRATORY PARTICLE DELIVERY TO THE NEXT CONDUIT GENERATION FOR DIFFERENT AIR AND PARTICLE FLOW RATES
602
GENERATE A REDUCED ORDER MODEL (ROM) INCLUDING TOTAL MASSES OF RESPIRATORY PARTICLES DEPOSITED IN THE GENERATIONS OF THE LUNG
604

FIG. 6

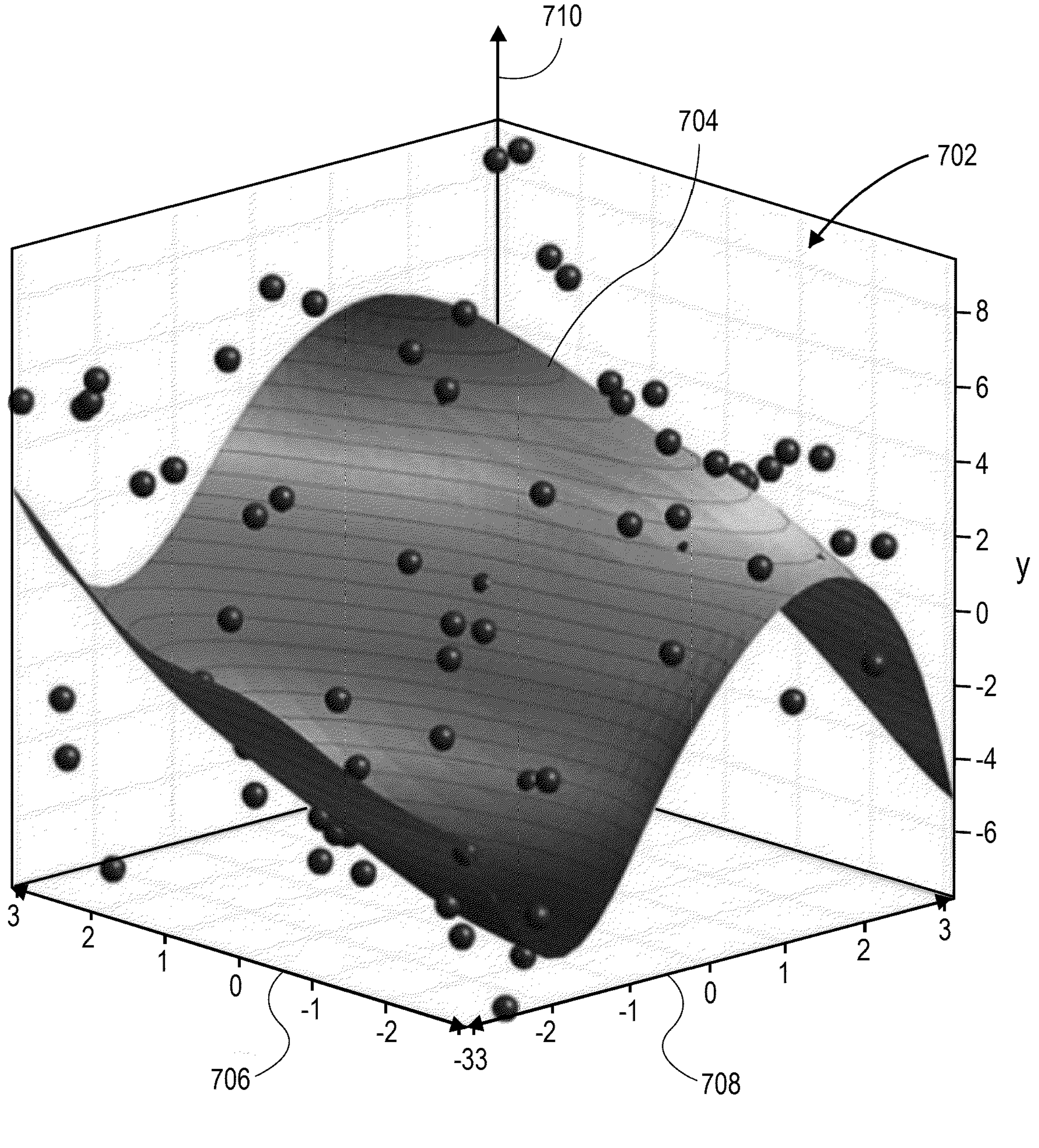


FIG. 7

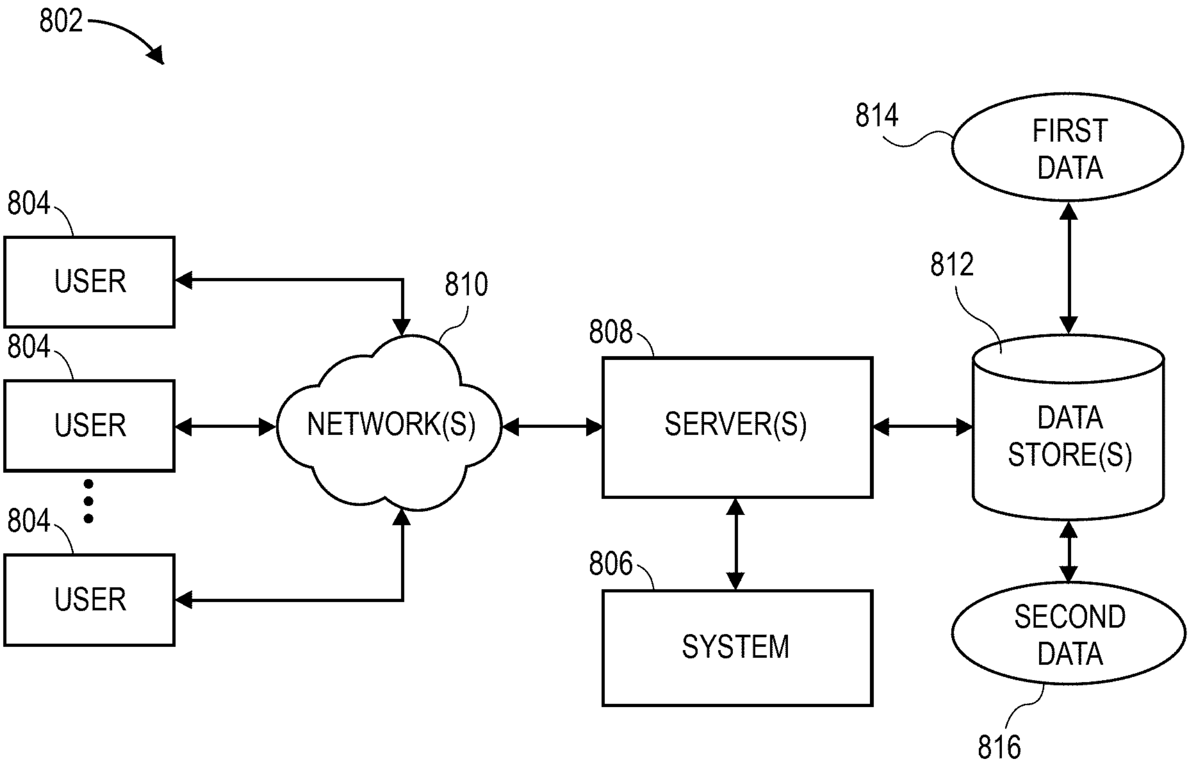
802
804
USER
804
USER
804
USER
810
NETWORK(S)
808
SERVER(S)
806
SYSTEM
814
FIRST DATA
812
DATA STORE(S)
SECOND DATA
816

FIG. 8

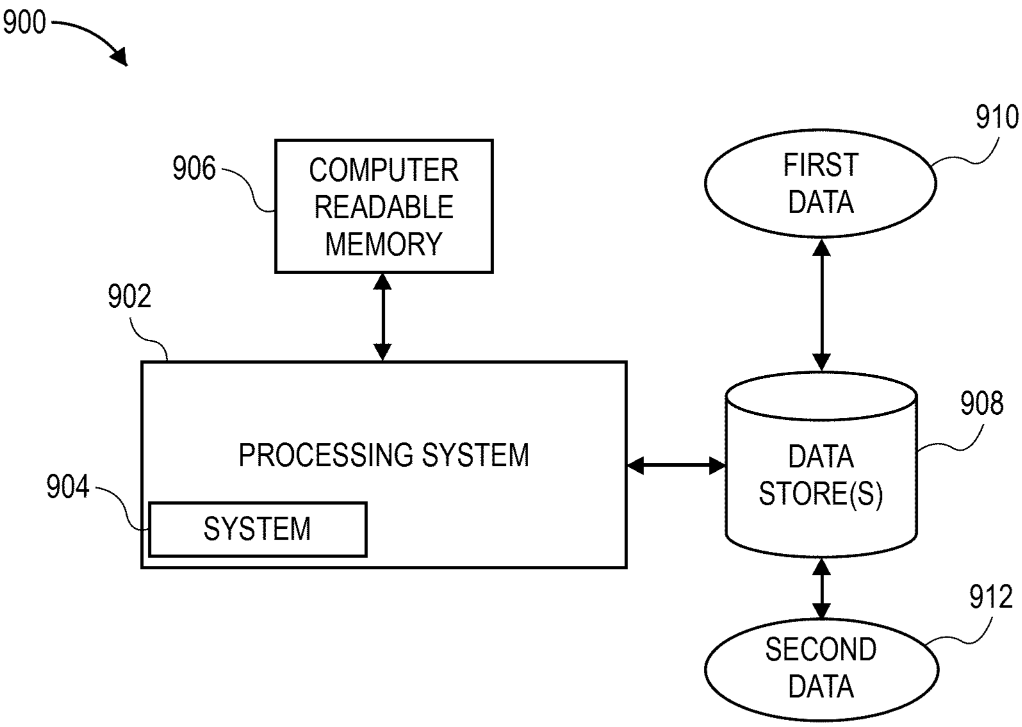
900
906
COMPUTER READABLE MEMORY
902
PROCESSING SYSTEM
904
SYSTEM
910
FIRST DATA
908
DATA STORE(S)
912
SECOND DATA

FIG. 9

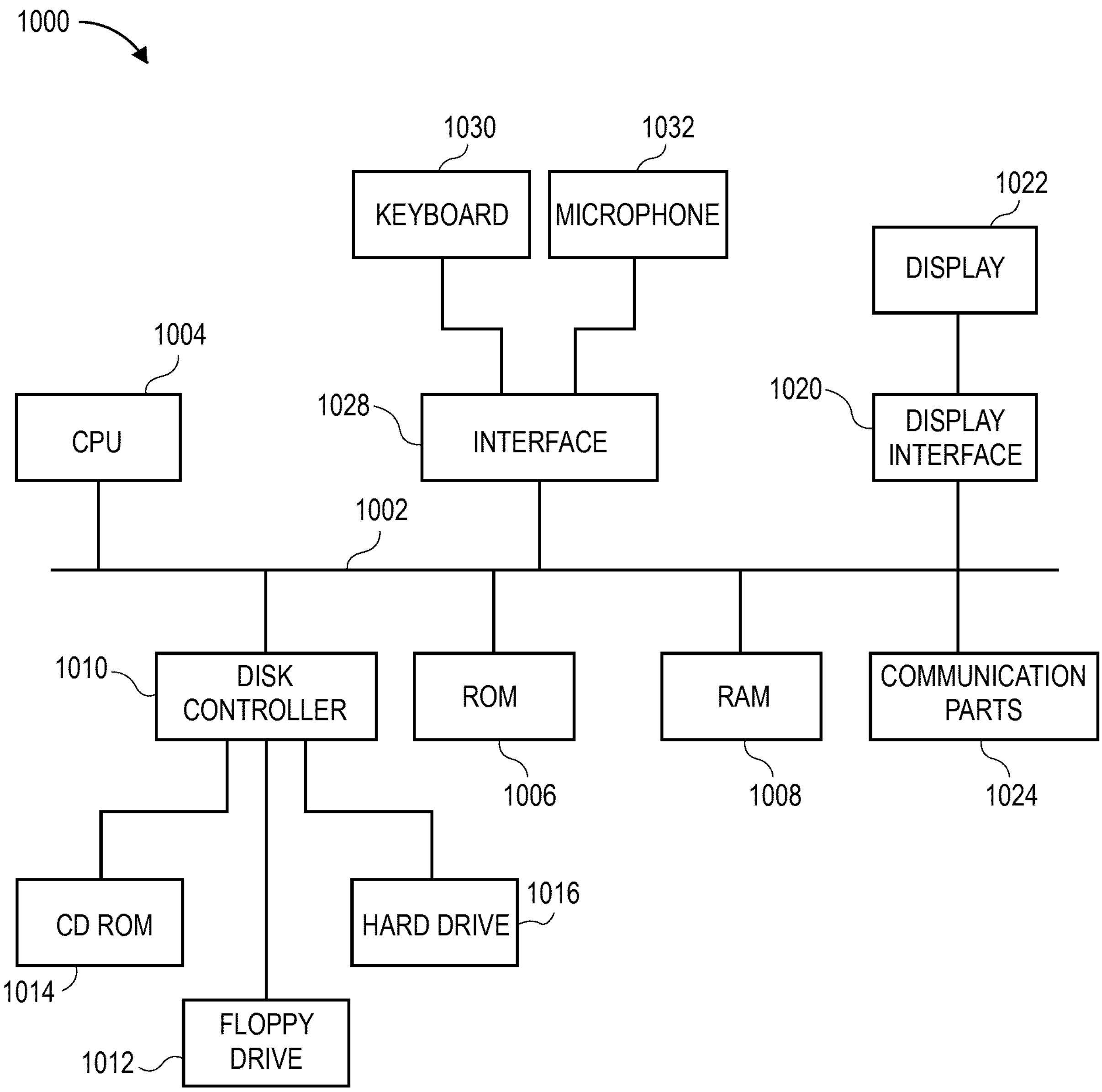
1000
1030
1032
KEYBOARD
MICROPHONE
1022
DISPLAY
1004
1028
INTERFACE
1020
DISPLAY INTERFACE
CPU
1002
1010
DISK CONTROLLER
ROM
RAM
COMMUNICATION PARTS
1006
1008
1024
1016
CD ROM
HARD DRIVE
1014
FLOPPY DRIVE
1012

FIG. 10

Europäisches Patentamt
European Patent Office
Office européen des brevets

# EUROPEAN SEARCH REPORT

Application Number
EP 22 19 3347

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YOUSEFI MORTEZA ET AL: "CFD simulation of aerosol delivery to a human lung via surface acoustic wave nebulization", BIOMECHANICS AND MODELING IN MECHANOBIOLOGY, SPRINGER BERLIN HEIDELBERG, BERLIN/HEIDELBERG, vol. 16, no. 6, 22 July 2017 (2017-07-22), pages 2035-2050, XP036353067, ISSN: 1617-7959, DOI: 10.1007/S10237-017-0936-0 [retrieved on 2017-07-22] * abstract; section "Introduction"; pages 2037, 2041, 2038; figures 2b, 11, 12, * ----- | 1-20 | INV. G16H20/10 G16H50/50 |

TECHNICAL FIELDS SEARCHED (IPC)

G16H

The present search report has been drawn up for all claims

| Place of search | Date of completion of the search | Examiner |
|---|---|---|
| Munich | 1 June 2023 | Wittke, Claudia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

3

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- IN 202241016187


### Non-patent literature cited in the description


- **MARTONEN et al.** Human Lung Morphology Models For Particle Deposition Studies. *Inhalation Toxicology,* 2000, vol. 12 (4), 109-121